# EUROPEAN PATENT APPLICATION

(11) **EP 3 335 624 A1**
(43) Date of publication of application: **20.06.2018**
(21) Application number: 16835502.2
(22) Date of filing: 12.08.2016
(51) Int. Cl.: A61B 5/00, A61B 6/00, G06F 3/0481, G06F 3/0485

(54) **GRAPHICAL USER INTERFACE PROVIDING METHOD FOR TIME-SERIES IMAGE ANALYSIS**

(30) Priority: 13.08.2015 KR 20150114911
(71) Applicant: Vieworks Co., Ltd., Anyang-si, Gyeonggi-do 14055 (KR)
(72) Inventor: KANG, You Jung, Anyang-si Gyeonggi-do 13928 (KR); KIM, Seung Gyu, Seoul 04775 (KR); KIM, Dae Hwan, Seongnam-si Gyeonggi-do 13475 (KR)
(74) Representative: Zacco Sweden AB
(86) International application number: PCT/KR2016/008939
(87) International publication number: WO 2017/026862

(57) **Abstract**

The present invention relates to a graphical user interface providing method for time-series image analysis, the method comprising the steps of: reading at least one time-series image set by a controller; displaying each read image set by the controller on each window; and providing a graphical user interface for receiving dynamic analysis configuration from a user by the controller.

## Description

### [Technical Field]

The present invention relates to a graphical user interface (GUI) providing method for time-series image analysis, and more specifically, to a GUI providing method for time-series image analysis which allows for analysis of time-series images captured over time.

### [Background Art]

Currently, single-photon emission computed tomography (SPECT), positron emission tomography (PET), angiography, and the like are being used to quantitatively measure blood perfusion in tissue.

Angiography is an examination method for diagnosing abnormality of a target tissue of a living body injected with a contrast agent by analyzing image data of energy emitted from the target tissue.

Although an imaging method of the angiography slightly differs depending on the type of contrast agent, the results of the angiography are the same in that each pixel of a captured image has only a value for a single indicator (e.g., intensity of light).

For example, indocyanine green (ICG) angiography utilizes the fact that indocyanine green absorbs near-infrared light of 730 to 790 nm and emits fluorescence in the near-infrared range of 800 to 850 nm, which is longer than a wavelength of the absorbed light. The ICG angiography measures the fluorescence using a CCD camera or a spectrometer to obtain an image. That is, the image captured by the ICG angiography corresponds to an image containing data according to the fluorescence intensity (e.g., an image having brightness proportional to the fluorescence intensity).

Meanwhile, a graphical user interface (GUI) on a computer system refers to an environment in which a user can work with graphics when exchanging information with a computer. That is, a GUI allows a user to easily recognize or manipulate information by displaying the information through a window, control icons, and the like.

Since an image captured through the above-described angiography is basically a very simple form, various types of data analysis have to be performed in order to analyze the image. Therefore, various GUI providing methods have been proposed to facilitate the data analysis.

However, since existing GUIs focus on analyzing a single image, they are unsatisfactory in analyzing time-series data, and thereby causing inconvenience to a user.

A background art of the present invention is disclosed in Korean Laid-Open Patent Publication No. 10-2007-0104520 (filed on October 26th, 2007).

### [Disclosure]

### [Technical Problem]

An objective of the present invention is to provide a graphical user interface (GUI) providing method for time-series image analysis which allows for easy analysis of time-sequentially formed image.

### [Technical Solution]

One aspect of the present invention provides a graphical user interface (GUI) providing method for time-series image analysis, including: reading, by a controller, at least one set of time-series images; displaying each set of image read by the controller on a window; and providing, by the controller, a GUI for receiving a dynamics analysis setting from a user.

The providing of the GUI may include: displaying, by the controller, a frame number of a currently displayed image; displaying, by the controller, an item for designating a section to be analyzed; and when the section to be analyzed is designated, changing, by the controller, a position of a pointer which indicates a start point and an end point of the scrollbar according to the designated section and displaying the pointer.

The providing of the GUI may include displaying, by the controller, a pointer which indicates a start point and an end point of the scrollbar and, when a position of the pointer is changed, changing, by the controller, a section to be analyzed according to the changed position of the pointer.

The providing of the GUI may include displaying, by the controller, an item for setting a time interval between frames of the images.

The providing of the GUI may include receiving, by the controller, a range of pixels to be analyzed among pixels of the images.

The receiving of the range of pixels may include receiving, by the controller, an analysis range through at least one of a pixel-based method, a region of interest (ROI)-based method, and a masking-based method.

The GUI providing method may further include displaying, by the controller, a graph of an image value of a selected pixel over time when the analysis range is input through the pixel-based method in the receiving of the range of pixels.

The providing of the GUI may further include displaying, by the controller, an item for setting whether to apply a regression analysis to the graph.

The providing of the GUI may further include displaying, by the controller, an item for setting normalization of the graph.

When the masking-based method is selected and a pixel is selected by the user in the receiving of the range of pixels, the controller may set pixels having image values whose difference with an image value of the selected pixel is less than a reference value as a mask.

When the reference value is changed, the controller may change an area of the mask according to the changed reference value, and the reference value may be changeable via scrolling a mouse wheel or a reference value setting item.

Two or more masks having different reference values may be settable, and an area of each mask may be changeable according to an input of the user.

The ROI may be shaped as one of a circle, an ellipse, a polygon, and a symmetrical form.

The GUI providing method may further include determining, by the controller, an analysis area to be analyzed on the basis of an ROI or a mask when the analysis range is input through the ROI-based method or the masking-based method in the receiving of the range of pixels and displaying, by the controller, a dynamics calculation result for the determined analysis area.

In the determining of the analysis area, when a mask exists, the controller may determine the mask area as the analysis area, and, when no mask exists, determine the ROI as the analysis area.

In the determining of the analysis area, when both a mask and an ROI exist, the controller may set a new ROI based on the mask and/or the ROI.

In the determining of the analysis area, when a plurality of ROIs exist, the controller may set pixels corresponding to an intersection between each of the ROIs and the mask as a plurality of new ROIs.

The displaying of the dynamics calculation result may include displaying, by the controller, a color map for the analysis area.

The displaying of the dynamics calculation result may include, when an ROI exists, displaying, by the controller, a feature value calculated within the ROI.

The displaying of the dynamics calculation result may include, when an ROI exists, displaying, by the controller, at least one of a maximum value, a minimum value, a mean value, and a standard deviation of a feature value calculated within the ROI.

The providing of the GUI may further include, when a plurality of ROIs are input, displaying, by the controller, an item for specifying a name of each of the ROIs in the receiving of the range of pixels.

The GUI providing method may further include storing information about an images read by the controller and data analyzed through the provided GUI, wherein in the storing of the information and the data, the controller stores the information about the images and the analyzed data in a form of a single file.

The information about the images may include at least one of information about the read images, a storage path of the images, and information about capturing conditions of the images.

### [Advantageous Effects]

A graphical user interface (GUI) providing method for time-series image analysis according to the present invention provides a GUI which allows time-sequentially formed images to be easily analyzed, thereby improving a user's convenience.

### [Description of Drawings]

FIG. 1 is an exemplary diagram for describing a graphical user interface (GUI) providing apparatus for time-series image analysis according to one embodiment of the present invention.
FIG. 2 is a flowchart for describing a GUI providing method for time-series image analysis according to one embodiment of the present invention.
FIG. 3 is an exemplary illustration for describing a way of displaying a plurality of time-series image sets in the GUI providing method for time-series image analysis according to one embodiment of the present invention.
FIG. 4 is an exemplary illustration for describing a way of displaying a scrollbar in the GUI providing method for time-series image analysis according to one embodiment of the present invention.
FIG. 5 is an exemplary illustration for describing a pixel-based dynamics analysis performed in the GUI providing method for time-series image analysis according to one embodiment of the present invention.
FIGS. 6A, 6B, 7A, and 7B are exemplary illustrations for describing a way of displaying a temporal position of a currently displayed time-series image in the GUI providing method for time-series image analysis according to one embodiment of the present invention.
FIGS. 8A, 8B, 9A, and 9 are exemplary illustrations for describing a way of changing a section to be analyzed in the GUI providing method for time-series image analysis according to one embodiment of the present invention.
FIG. 10 is an exemplary illustration for describing a way of simultaneously comparing a plurality of time-series image sets with each other in the GUI providing method for time-series image analysis according to one embodiment of the present invention.
FIGS. 11A and 11B are exemplary illustrations for describing a way of setting a time interval between frames of a time-series image in the GUI providing method for time-series image analysis according to one embodiment of the present invention.
FIGS. 12A and 12B are exemplary illustrations for describing an application of a regression analysis to a signal change graph over time in the GUI providing method for time-series image analysis according to one embodiment of the present invention.
FIGS. 13A and 13B are exemplary illustrations for describing an application of normalization to a signal change graph over time in the GUI providing method for time-series image analysis according to one embodiment of the present invention.
FIG. 14 is an exemplary illustration for describing a way of setting masking in the GUI providing method for time-series image analysis according to one embodiment of the present invention.
FIGS. 15 and 16 are exemplary illustrations for describing a way of setting a region of interest (ROI) in the GUI providing method for time-series image analysis according to one embodiment of the present invention.
FIGS. 17 and 18 are exemplary illustrations for describing a way of performing dynamics analysis in the GUI providing method for time-series image analysis according to one embodiment of the present invention.
FIG. 19 is an exemplary illustration for describing a dynamics analysis through an ROI and masking in the GUI providing method for time-series image analysis according to one embodiment of the present invention.
FIGS. 20A and 20B are exemplary illustrations for describing a way of changing a color map in the GUI providing method for time-series image analysis according to one embodiment of the present invention.
FIGS. 21A, 21B, 21C, 22A, and 22B are exemplary illustrations for describing a way of sending out an analysis result in the GUI providing method for time-series image analysis according to one embodiment of the present invention.
FIGS. 23A and 23B are exemplary illustrations for describing a way of setting a name of an ROI in the GUI providing method for time-series image analysis according to one embodiment of the present invention.

### [Modes of the Invention]

Hereinafter, one embodiment of a graphical user interface (GUI) providing method for time-series image analysis according to the present invention will be described with reference to the accompanying drawings. In the drawings, thicknesses of lines or relative sizes of elements may be exaggerated for clarity and convenience of illustration. In addition, some terms described below are defined in consideration of functions in the present invention, and meanings thereof may vary depending on, for example, a user or operator's intention or custom. Therefore, definitions of the terms should be made on the basis of the overall context of the specification.

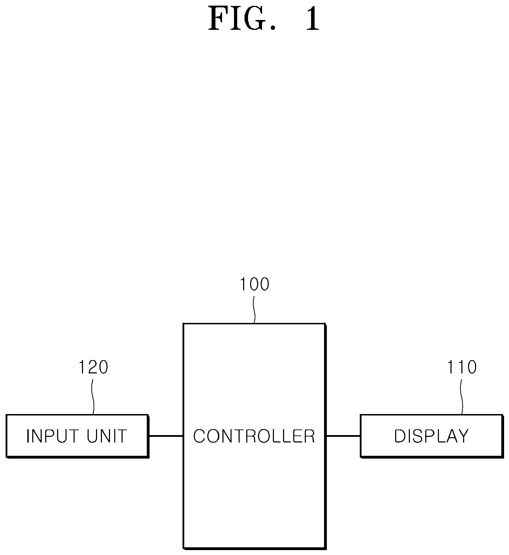

As shown in FIG. 1, a GUI providing apparatus for time-series image analysis according to one embodiment of the present invention includes a controller 100, a display 110, and an input unit 120.

The display 110 may display a GUI. For example, the display 110 may be a monitor of a computer system.

The input unit 120 may receive an interface control, such as a menu selection, an icon operation, and the like, from a user. The input unit 120 may be a keyboard, a mouse, a touchscreen, or the like of a computer system.

The controller 100 may provide the GUI through the display 110, and may process and analyze a time-series image, that is, data for a time-sequentially formed image. In the present invention, the time-sequentially formed image may be represented as a time-series image set, and the time-series image set may be a general image file, for example, a set of a number of images in a jpg, tif, or the like format that are captured over time, or a file captured by a time-series image, for example, one image file captured in an avi format. In this case, the time-series image may be an in-vivo image.

That is, the GUI providing apparatus for time-series image analysis according to one embodiment of the present invention may be a general computer system.

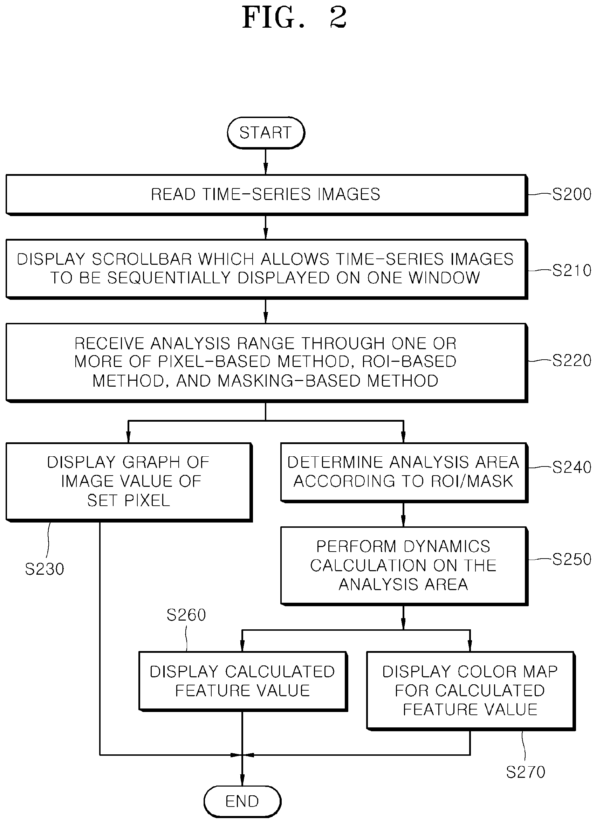

FIG. 2 is a flowchart for describing a GUI providing method for time-series image analysis according to one embodiment of the present invention. The GUI providing method for time-series image analysis according to the embodiment will be described with reference to FIG. 2 below.

As shown in FIG. 2, the controller 100 first reads time-series images (S200). In this case, the time-series images refer to a plurality of images formed into one set. The images may be in-vivo images. That is, time-series in-vivo images may refer to images of an in-vivo region of interest (ROI) taken a number of times over time (e.g., 120 images obtained by capturing an image per second for 2 minutes).

The time-series in-vivo images may be images in which objects are distinguished only by brightness. That is, data stored in each pixel of the time-series in-vivo image may contain only a brightness value, and a difference between brightness values may be information indicating a state of an object to be imaged.

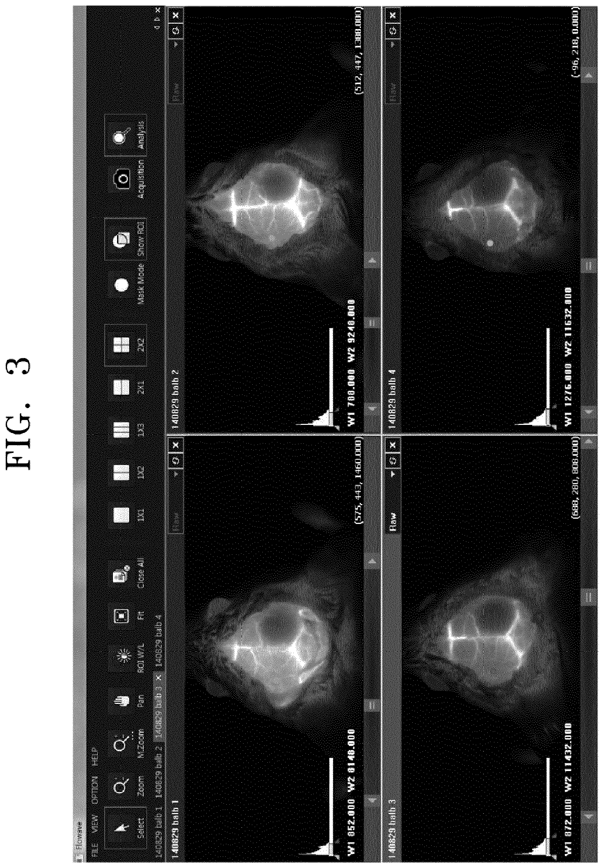

In operation S200, the controller 100 may not read a single image set, but may read a plurality of image sets, and, as shown in FIG. 3, images included in each of the sets may be displayed on a window assigned to each set.

In addition, the time-series images may be captured in advance and stored in a storage device, such as a hard disk drive.

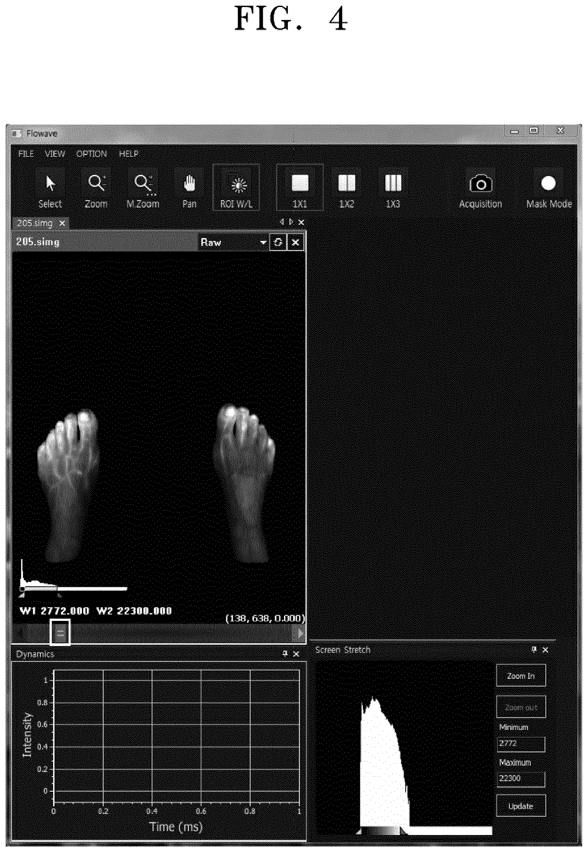

Then, the controller 100 displays a scrollbar which allows the time-series images to be sequentially displayed on one window (S210). That is, as shown in FIG. 4, the controller 100 may display a scrollbar which indicates a temporal position of an image currently displayed and the time-series images, and may sequentially change the displayed image according to movement of the scrollbar.

In other words, a user may move the scrollbar left and right or up and down to view images before or after the current image in order.

In this case, when the user changes a setting (e.g., brightness) of one image, the controller 100 may apply the same setting to all of the time-series images in which a pertinent image is included.

After operation S210, the controller 100 receives an analysis range setting from the user through one or more of methods including a pixel-based method, a ROI-based method, and a masking-based method (S220).

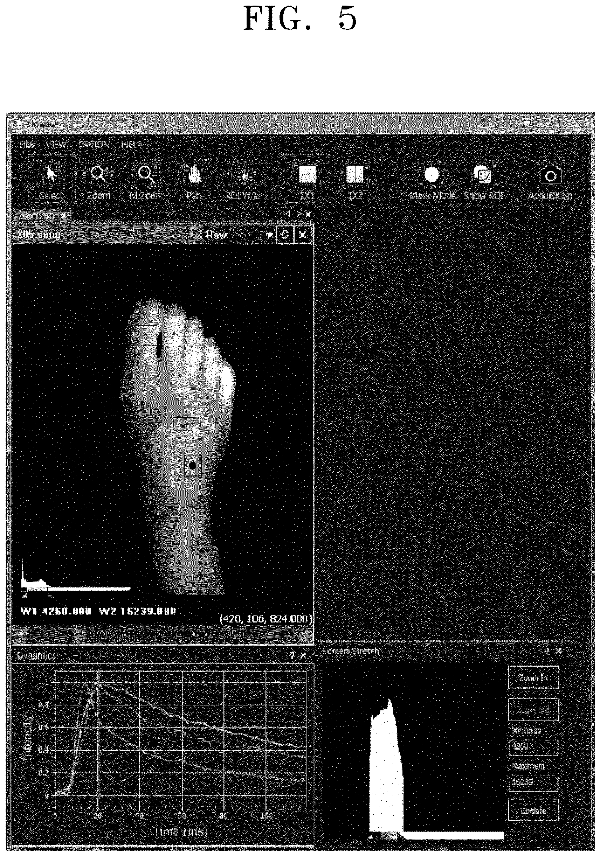

The pixel-based method refers to a method of designating specific pixels as an analysis range. That is, as can be seen in FIG. 5, the user may set the pixel-based method as the analysis range setting method through the input unit 120, and select one or more pixels (3 pixels in FIG. 5) as the analysis range. In this case, when a plurality of pixels are selected, the selected pixels may be displayed by dots of different colors.

When the user selects the analysis range through the pixel-based method, the controller 100 may analyze signal changes over time within the selected analysis range.

That is, when the analysis range is set through the pixel-based method in operation S220, the controller 100 displays a graph of an image value according to a time of the set pixels (S230). In this case, the image value indicates data (e.g., brightness) stored in a corresponding pixel. That is, as can be seen in FIG. 5, signal changes over time for the three pixels may be provided in the form of a graph. At this time, colors of the displayed graphs may be the same as the colors of the corresponding dots.

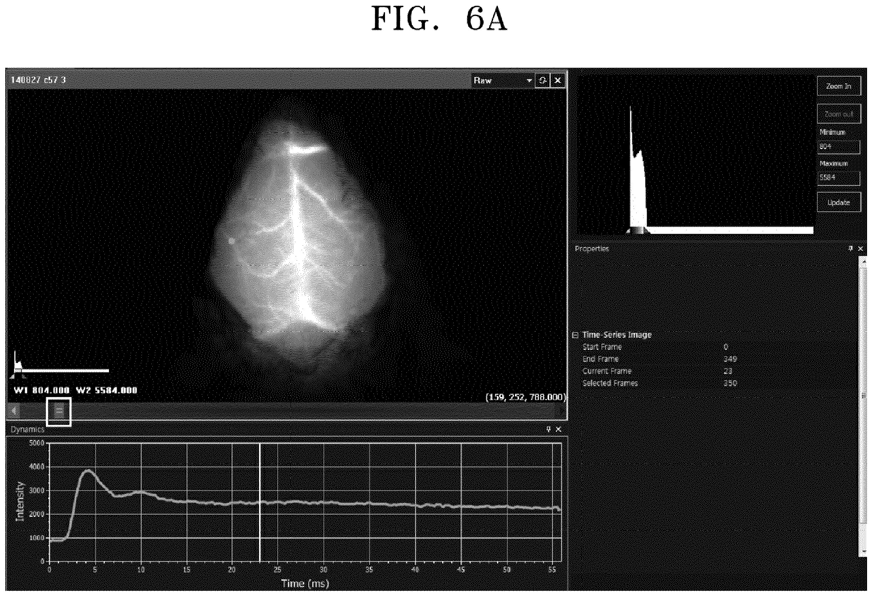

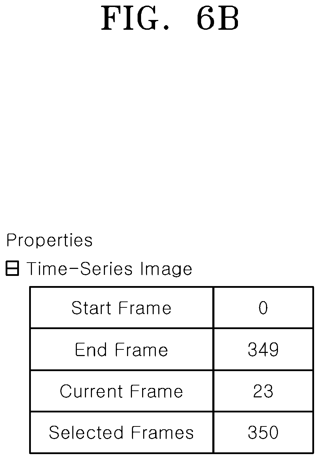

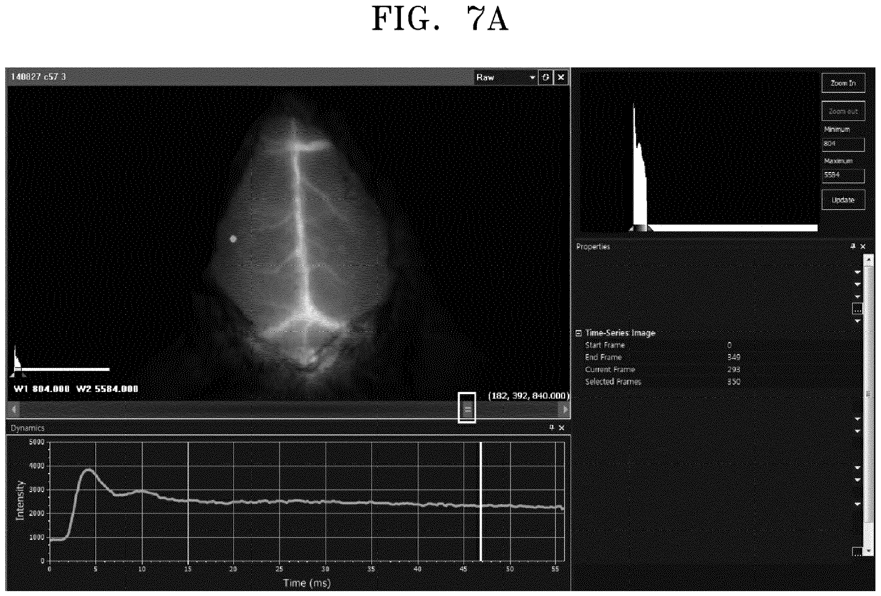

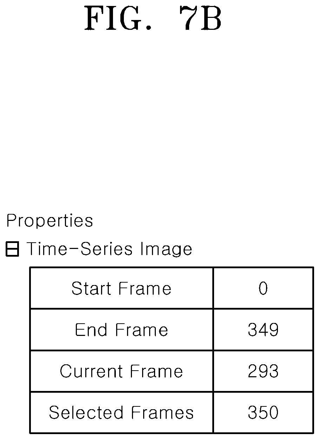

In addition, as can be seen in FIGS. 6A and 6B (FIG. 6B is an enlarged view of an area of displaying properties of FIG. 6A) and FIGS. 7A and 7B (FIG. 7B is an enlarged view of an area of displaying properties of FIG. 7A), the controller 100 may display a frame number of the currently displayed time-series image in addition to the scrollbar, and may display a position bar (represented as a bar in FIGS. 6A, 6B, 7A, and 7B) for identifying a temporal position of the currently displayed image in the signal change graph over time.

Accordingly, the user may identify a frame number which is not needed for the analysis while browsing back and forth through the time-series images. The time-series images are captured in such a manner that a contrast agent is injected after starting the capturing of the images to acquire data from a time point at which the contrast agent is injected. In other words, such time-series images include images captured before the injection of the contrast agent, and it may be desirable for such images to be excluded because they are not useful in analysis. Therefore, displaying the frame number, as shown in FIGS. 6A, 6B, 7A, and 7B, may enable the user to easily identify unnecessary data.

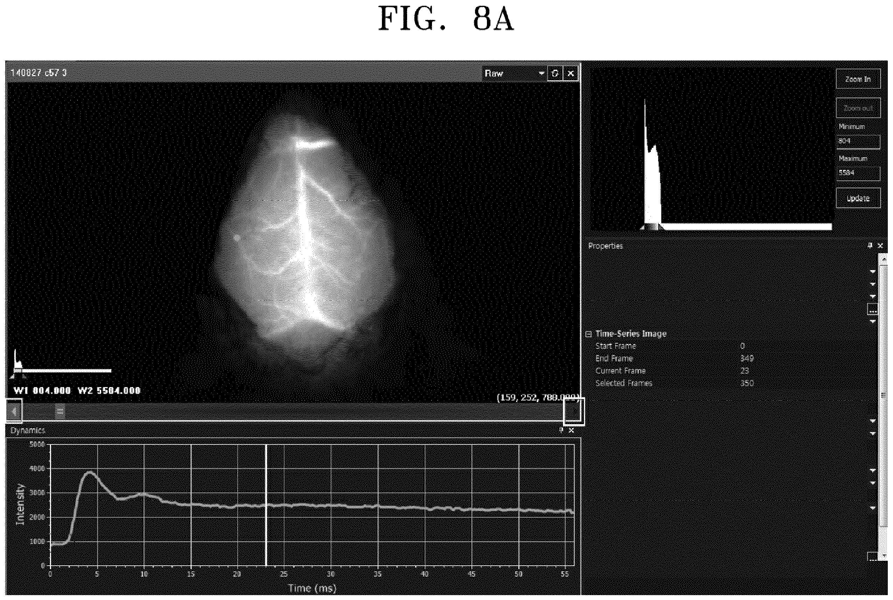

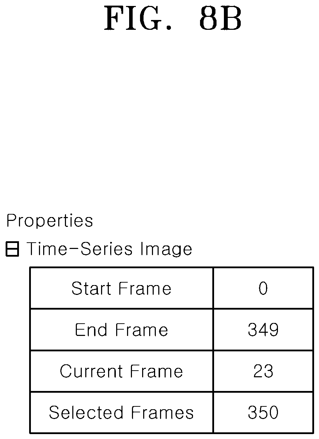

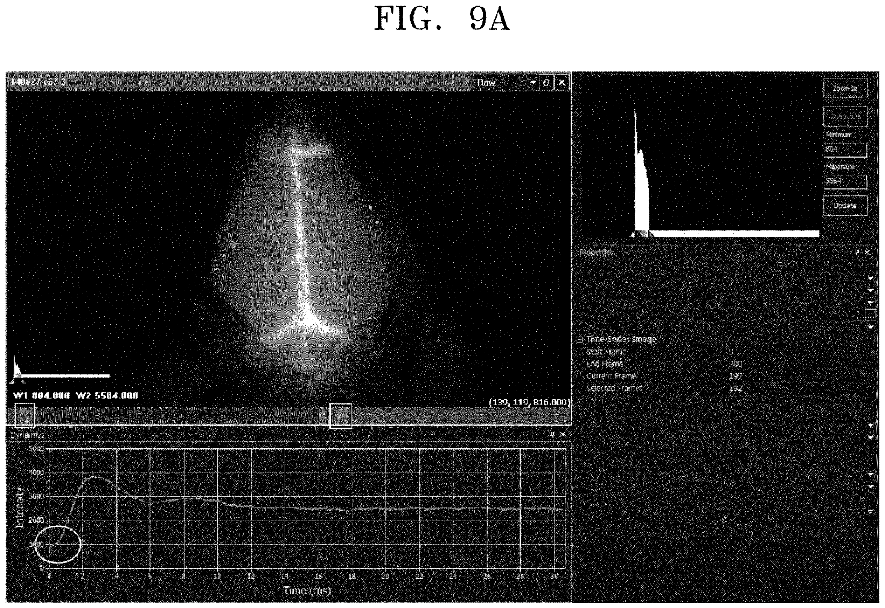

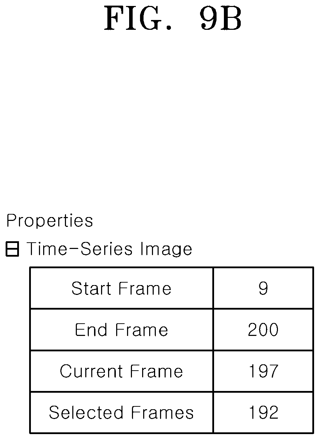

In addition, as can be seen in FIGS. 8A and 8B, (FIG. 8B is an enlarged view of an area of displaying properties of FIG. 8A) and FIGS. 9A and 9B (FIG. 9B is an enlarged view of an area of displaying properties of FIG. 9A), the controller 100 may display items for designating a start point and an end point for performing the analysis. That is, the user may designate a section on which the analysis is to be performed by inputting a desired frame number into a "Start Frame" item and an "End Frame" item.

When the section to be analyzed is changed as described above, the controller 100 may automatically change a position of a pointer indicating a start and end of the scrollbar according to the changed section and display the pointer.

In addition, the pointer displayed by the controller 100 may be in a form capable of being autonomously moved. That is, the user may designate the section to be analyzed by moving the pointer as well as by inputting the frame numbers. For example, the user may change the position of the pointer by clicking and dragging the pointer. When the position of the pointer is moved as described above, the controller 100 may automatically change and display the numbers indicated in the "Start Frame" item and the "End Frame" item.

Also, as described above, when the section to be analyzed is changed, the controller 100 may change a displayed section of the signal change graph over time according to the section to be analyzed. Excluding data unnecessary for the analysis by changing the section to be analyzed improves the accuracy of analysis.

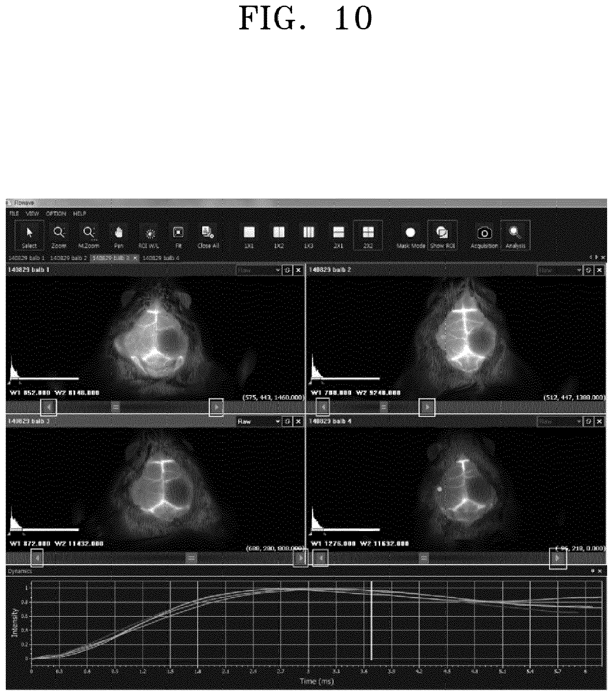

Meanwhile, as can be seen in FIG. 10, when a plurality of time-series image sets are simultaneously displayed, the controller 100 may display the signal change graphs according to time for each of the pixels in one window.

Generally, dynamics analysis using laboratory animals involves imaging, analyzing, and compiling statistics using one or more laboratory animals. Hence, when a plurality of time-series images are simultaneously displayed while signal changes over time for a pixel selected from each of the time-series images are displayed on a single graph as in the present embodiment, comparative analysis among a number of experimental animals may be easily performed.

In this case, since capturing conditions (e.g., a time for injecting a contrast agent) may be different for each set, adjusting the each of the sections to be analyzed as in the present embodiment may make it possible to more accurately compare the signal changes over time to each other.

That is, since a degree of deviation of control dynamics among the experimental animals may be identified through the above-described procedures, the GUI providing method for time-series image analysis according to the present invention may allow the user to exclude data of an experimental animal having a large deviation in its tissue in advance.

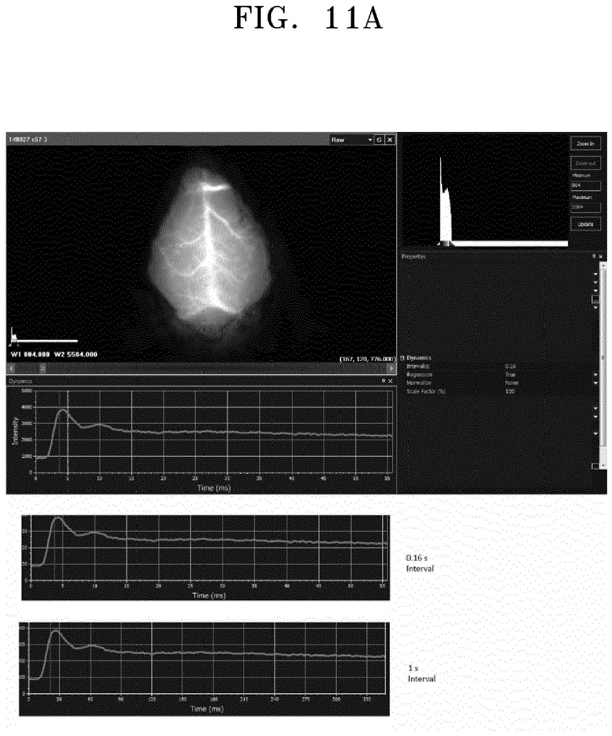

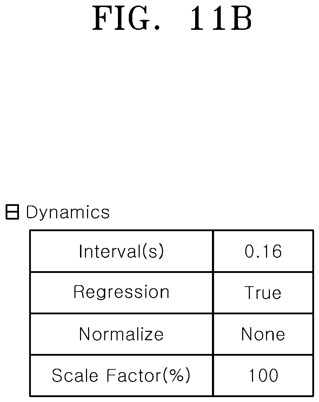

In addition, as can be seen in FIGS. 11A and 11B (FIG. 11B is an enlarged view of an area of displaying properties of FIG. 11A), the controller 100 may display an item for setting a time interval between frames of the time-series images. That is, the user may increase accuracy of analysis by inputting an actual interval at which the time-series images are imaged to an "Interval(s)" item.

When the time interval of the time-series images is changed, the controller 100 may automatically adjust and display an x-axis (time axis) of the signal change graph over time.

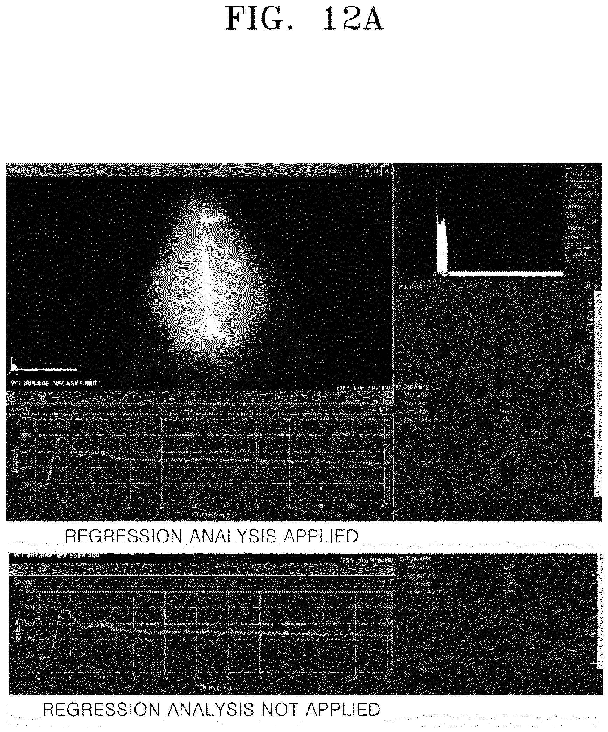

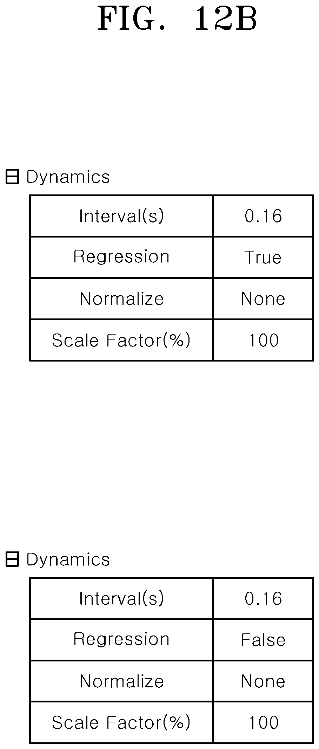

In addition, as can be seen in FIGS. 12A and 12B (FIG. 12B is an enlarged view of an area of displaying properties of FIG. 12A), the controller 100 may display an item for a setting of determining whether to apply a regression analysis to the signal change graph over time. That is, the user may select whether to apply the regression analysis to the signal change graph over time by selecting either "True" or "False" for a "Regression" item.

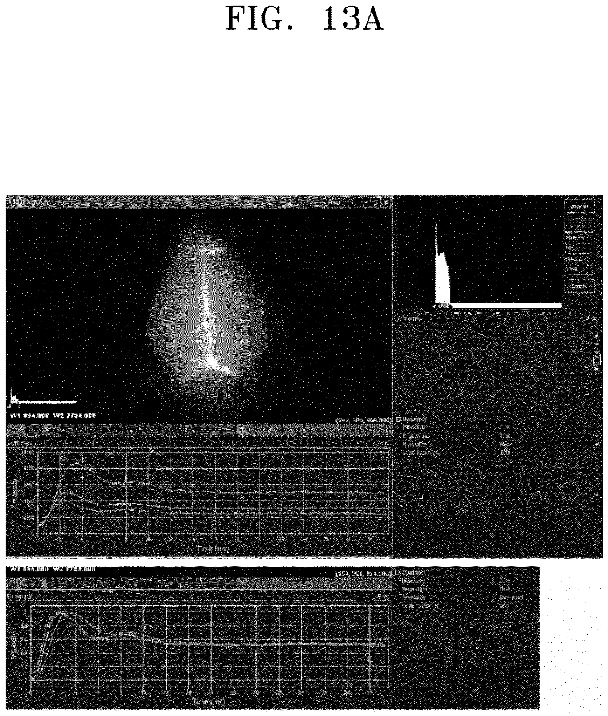

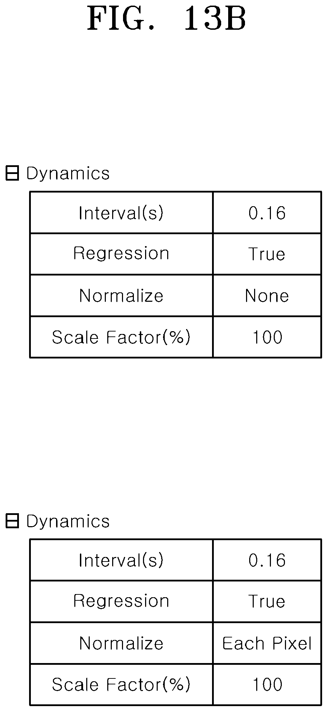

Moreover, as can be seen in FIGS. 13A and 13B (13B is an enlarged view of an area of displaying properties of FIG. 13A), the controller 100 may display an item for setting normalization of the signal change graph over time. In this case, there may be three options for the normalization setting item. The first option is for performing no normalization, the second option is for performing normalization on each pixel, and the last option is for performing normalization based on the highest image value within the whole analysis range.

That is, the user may select the normalization of the signal change graph over time by selecting one of "None," "Each Pixel," and "Whole Frame" for a "Normalize" item.

Meanwhile, among the above described GUI providing method in the operation S230, displaying the frame number of the currently displayed time-series image, displaying the pointer, changing the section to be analyzed, and setting the time interval between the frames may be performed not only in operation S230, but also in operation S220, or operations S240 to S270, which will be described below.

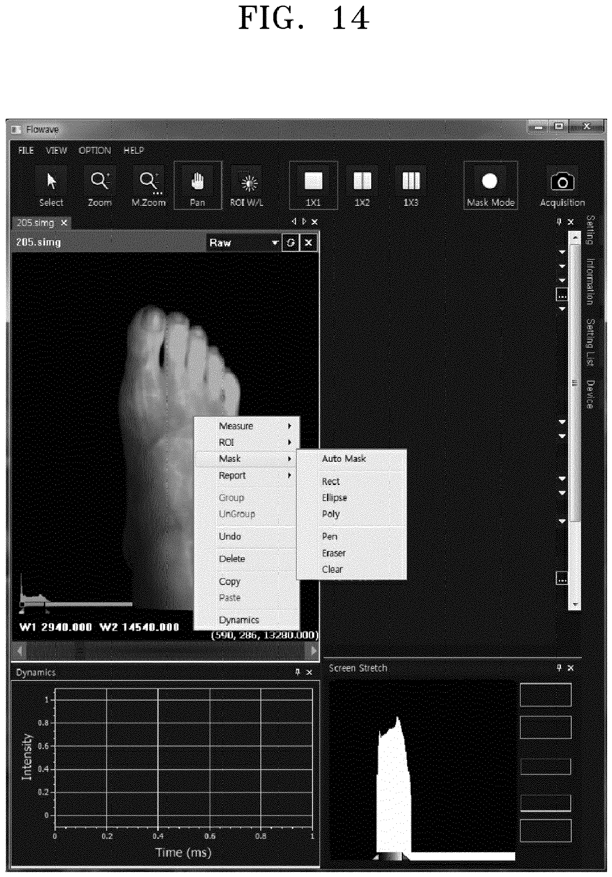

Meanwhile, in operation S220, the masking-based method selects only an in vivo form except for a background on the basis of the image value of the image. That is, for example, when the user specifies an auto-mask method as the analysis range setting method and selects a representative pixel through the input unit 120, the controller 100 may select adjacent pixels of the selected pixel which have image values similar to that of the selected pixel as a mask (a foot-shaped portion in FIG. 14), as shown in FIG. 14. When the masking-based method is used, a portion other than the mask is recognized as the background, and the analysis is not performed thereon so that an overall analysis time can be improved. Meanwhile, only one pixel may be selected as the mask according to a reference value.

One or more masks may be formed, and the controller 100 may provide a GUI for setting a selection range of masking (i.e., a setting range of a difference in image values between the pixel selected by the user and pixels automatically selected by the controller 100).

For example, a reference value may be changed through scrolling a mouse wheel or through a reference value setting item, and when the reference value is changed, the controller 100 may automatically change the mask area according to the changed reference value.

In addition, two or more masks having different reference values may be set, and an area of each of the masks may be changed according to the user's input. For example, a part of the mask area may be deleted from or added to an original mask area according to the user's input.

The ROI-based method is a method in which the user arbitrarily selects an ROI. That is, the user may specify the ROI-based method as the analysis range setting method through the input unit 120 and arbitrarily set an ROI (or only one pixel) to be analyzed.

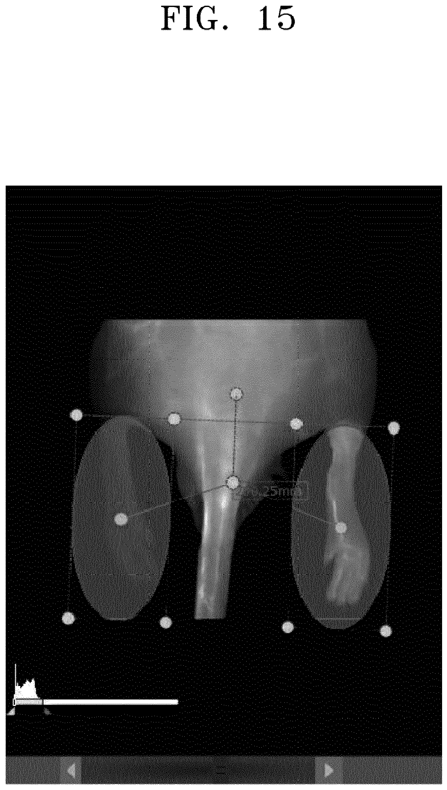

In this case, a plurality of ROIs may be set, and each of the ROIs may be shaped as one of a circle, an ellipse, a polygon, a free form, and a symmetrical form. That is, as shown in FIG. 15, the ROI may be a symmetrical form, and the symmetrical form means same shape on both of left and right sides of the center line. In other words, when the symmetrical ROI-based method is specified as the analysis range setting method, the controller 100 may automatically generate a symmetrical ROI even when the user sets only one ROI.

Accordingly, the symmetrical ROI may improve the user's convenience of analysis because the in vivo form is generally bilaterally symmetrical and there are many experiments that control only one side of the left and right sides and compare a reaction of the opposite side.

In addition, as can be seen in FIG. 15, the controller 100 may provide a GUI for moving a position of a center line of a symmetrically-shaped ROI or rotating the ROI with respect to one point on the center line.

Further, when a plurality of time-series image sets are displayed, the controller 100 may provide a GUI for copying and pasting an ROI generated on one of the time-series images on different time-series images. That is, it is possible to automatically generate ROIs with the same size at the same positions of the respective time-series images to perform comparative analysis on the plurality of time-series images.

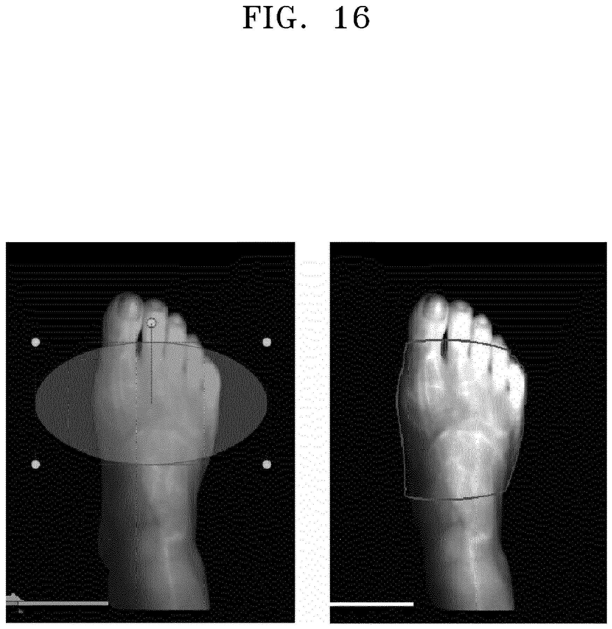

Meanwhile, as can be seen in FIG. 16, when both a mask and an ROI are set, the controller 100 may set only an intersection between the mask and the ROI as a new ROI. That is, since a part other than the mask can be considered as the background, excluding such a part from the ROI may improve speed of analysis.

In addition, when a plurality of ROIs exist, the controller 100 may set pixels corresponding to the intersections between each of the ROIs and the mask as a plurality of new ROIs.

As such, when the analysis range is set through the ROI or masking-based method in operation S220, the controller 100 determines an analysis area on which dynamics analysis is to be performed according to the presence of the ROI or mask (S240). For example, when masking is performed and a mask exists in operation S220, the controller 100 may determine the mask area to be the analysis area. That is, as described above, since the range of the ROI cannot deviate from the mask area when the mask exists, once the mask area is determined as the analysis area, the ROI is also included in the analysis area. Conversely, when no mask exists (i.e., when only the ROI exists), the controller 100 may determine the ROI as the analysis area.

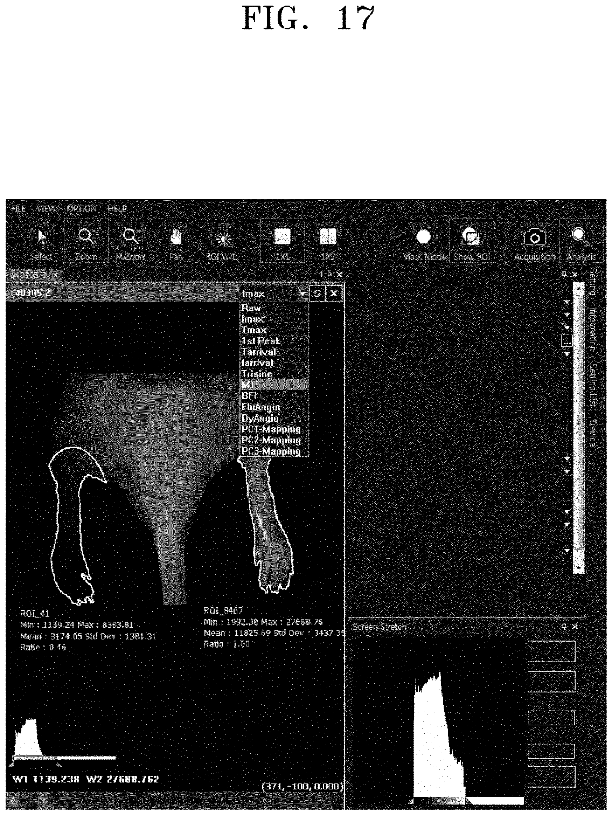

Then, the controller 100 performs dynamics calculation on the analysis area determined in operation S230 (S250). As shown in FIG. 17, the controller 100 may calculate various feature values, such as a differential value or integral value at specific time, a time value having a specific image value, principal component analysis (PCA), mean transit time (MTT), blood flow index (BFI), Tmax, and the like, which can be extracted by the dynamics analysis over time. However, the method of calculating these feature values corresponds to a technique already known in the technical field of the present invention, and thus a detailed description thereof will be omitted.

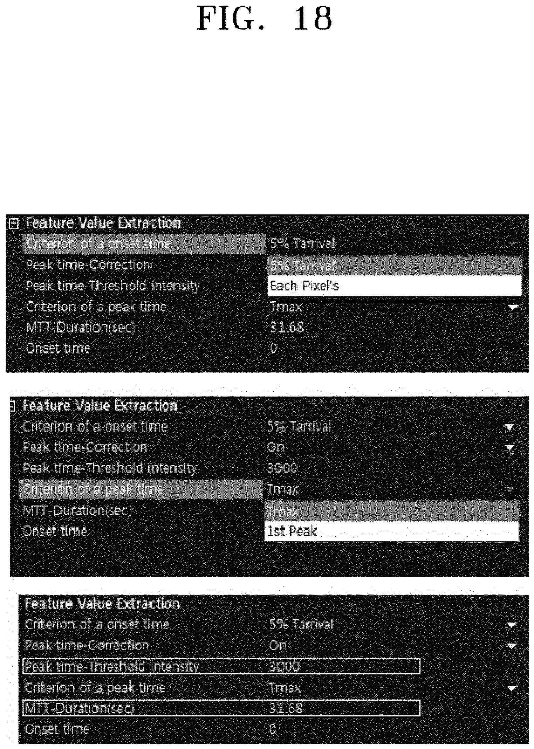

In addition, as can be seen in FIG. 18, the controller 100 may display items for setting variables for the dynamics calculation. In this case, an "Onset time" setting is a reference for determining an image in which an initial signal (an image value and intensity) is shown, "5% Tarrival" indicates a method of analyzing a time point at which a signal appears in 5 % of the entire ROI as a beginning of the signal, and "Each Pixel" indicates a method of individually analyzing start points of signals of respective pixels.

In addition, a "Criterion of a peak time" item is a variable for determining a peak time of an image value, "Tmax" indicates a method of determining a time of the highest peak as a peak time when there are a number of peaks on a graph, and "1^{st} peak" indicates a method of determining a time of the first peak as a peak time.

A "Peak time-threshold intensity" item is an item for removing noise data. When the maximum image value (brightness) of a pixel to be analyzed is less than a threshold set in the item, the controller 100 does not analyze the pixel. That is, the controller 100 removes noise data to reduce a time spent on the analysis.

"MTT-Duration" indicates a time interval for calculating an MTT. That is, since the MTT varies according to the MTT-duration, "MTT-Duration" item may be provided to analyze a plurality of images at the same duration.

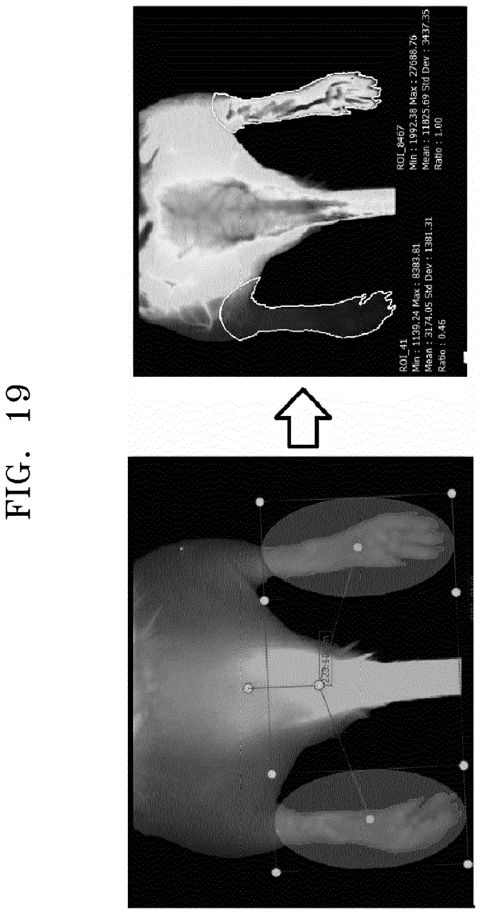

After operation S250, the controller 100 displays the feature values calculated in operation S250 (S260). For example, the controller 100 may display only feature values calculated within the ROI. That is, as can be seen in FIG. 19, when both the mask and the ROI are set, only an intersection between the mask and the ROI is determined as a new ROI, as described above, and the controller 100 may display feature values calculated within the new ROI. In this case, the controller 100 may display the maximum values, minimum values, mean values, and standard deviation of the feature values as well as the feature values.

In addition, after operation S250, the controller 100 displays a color map for the feature values calculated in operation S250 (S270). For example, the controller 100 may display a color map within the mask area. The color map may be displayed in the entire mask area as well as in the ROI. This method can reduce the time required to analyze data since the method allows dynamics calculation results for a tissue other than a target tissue to be viewed at a glance and simultaneously obtains feature values of only the target tissue. Meanwhile, when no mask exists, the controller 100 may display a color map within the ROI.

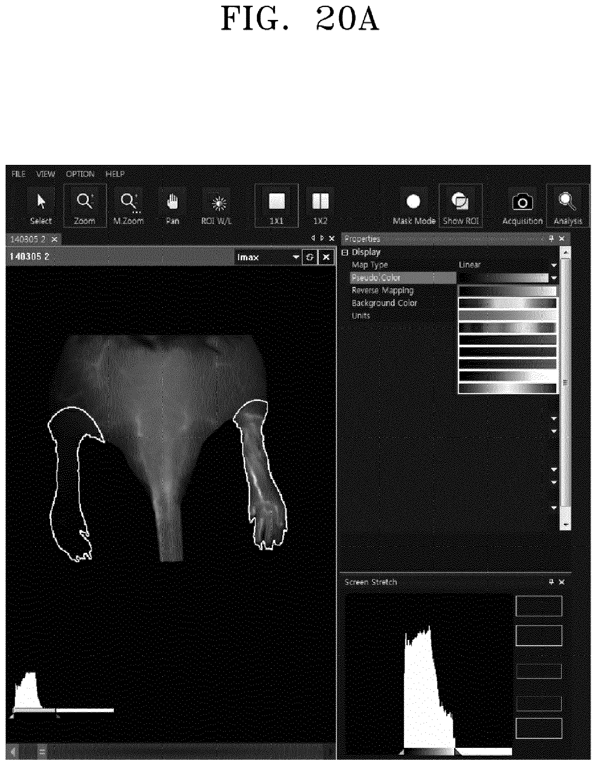

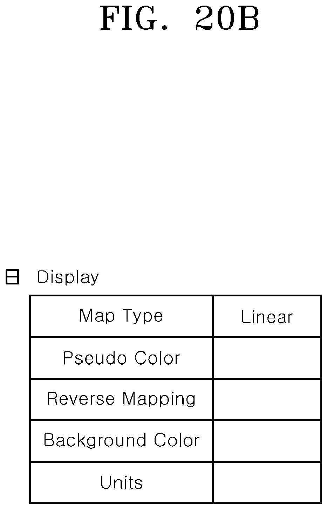

Further, as can be seen in FIGS. 20A and 20B (FIG. 20B is an enlarged view of an area of displaying properties of FIG. 20A), the controller 100 may display an item for setting a color configuration of the color map. That is, the user may determine the color configuration of the color map by selecting one of a number of color bars.

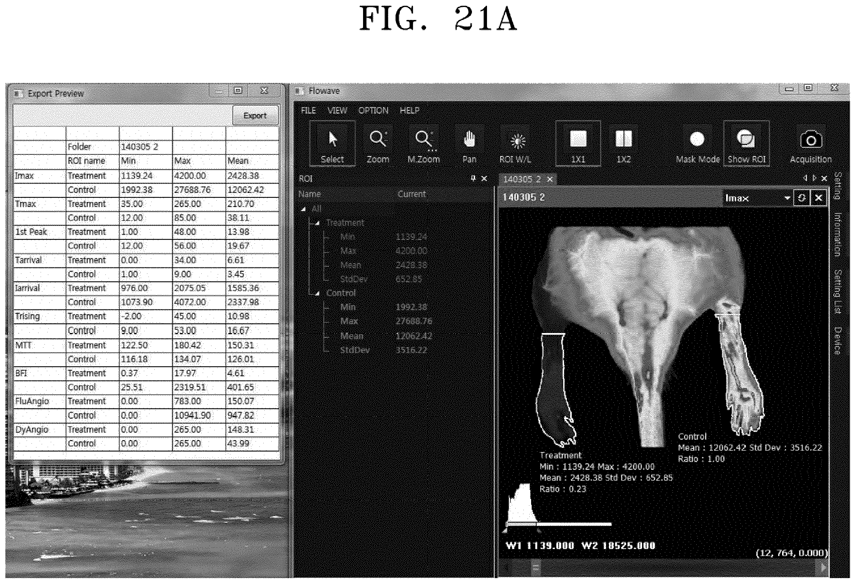

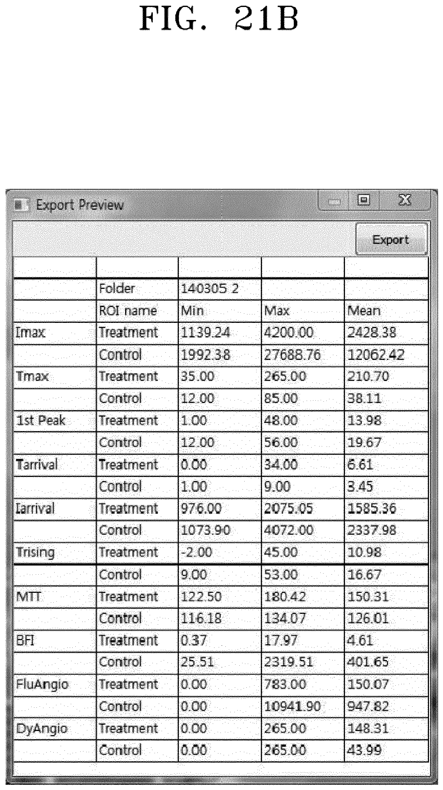

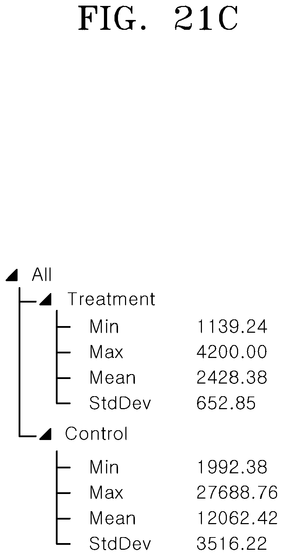

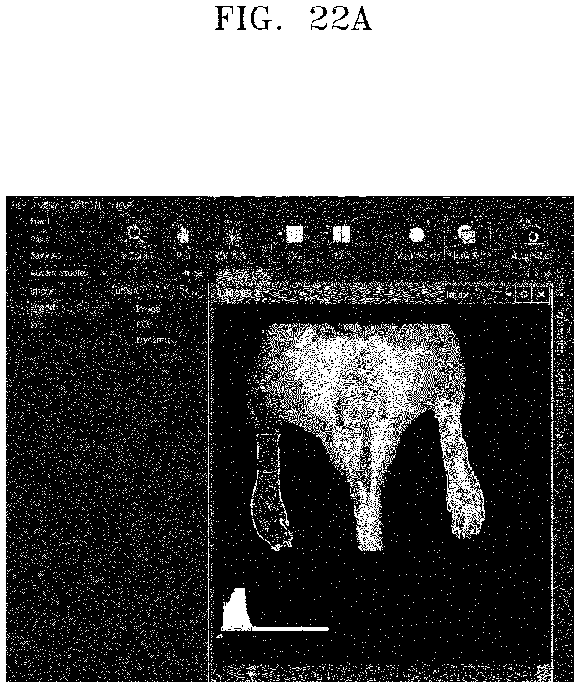

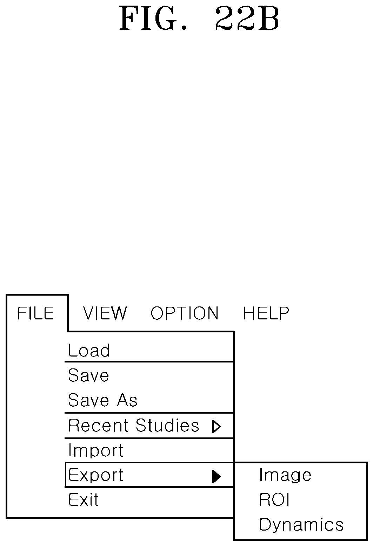

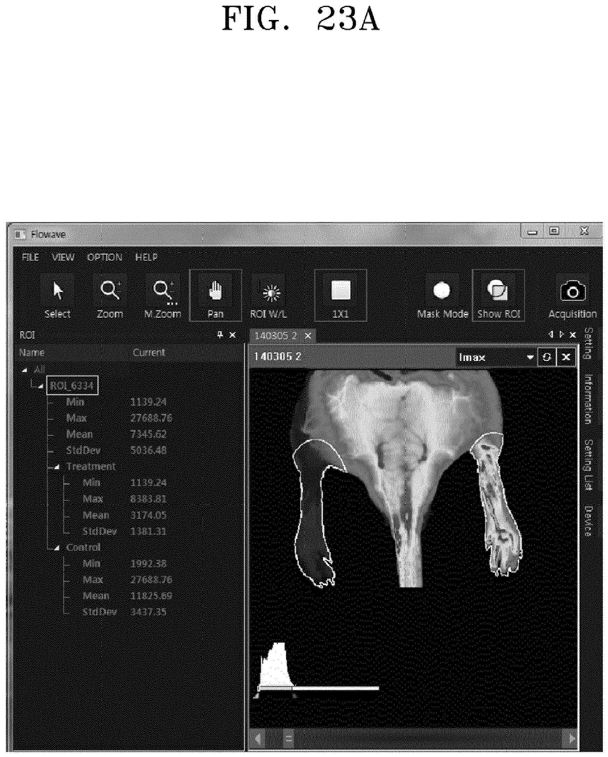

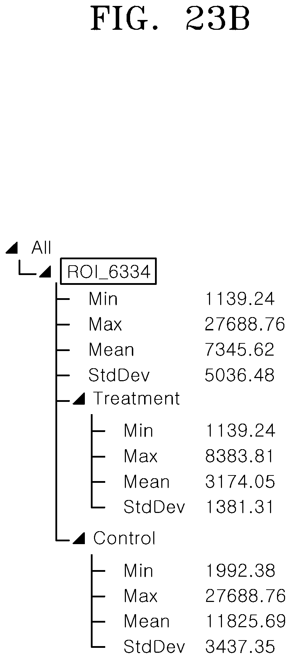

Meanwhile, as can be seen in FIGS. 21A, 21B, and 21C (FIG. 21B is an enlarged view of the left table of FIG. 21A, and FIG. 21C is an enlarged view of a feature value portion in the right screen of FIG. 21A) and FIGS. 22A and 22B (FIG. 22B is an enlarged view of a menu portion of FIG. 22A), the controller 100 may provide a GUI for sending calculated feature values, a color map, and a signal change graph over time for a specific pixel in the form of a table or an image file. In this case, since the feature values for the plurality of ROIs may be simultaneously sent, as can be seen in FIGS. 23A and 23B (FIG. 23B is an enlarged view of a feature value portion of FIG. 23A), the controller 100 may display items for specifying names of the ROIs.

In addition, data generated during the analysis, such as information on the read time-series image sets, information on the section to be analyzed, information on the range of pixels (masking, ROI, etc.) to be analyzed, the color map, and a graph of image values of a pixel over time, may be stored in the controller 100 in the form of a single file.

Accordingly, the user may check the analysis results later by reading the stored data, and may perform a new analysis process or a continuous analysis process such as changing the previous analysis conditions.

In this case, information about the image may include at least one of the image itself, a storage path of the image, and information about capturing conditions of the image. That is, when the image set is stored with the information, storage space taken by the image set and the information may be excessively increased, and therefore only information about the storage location of the read time-series image and the analyzed data are stored, and, when the stored file is read later, the image stored at the corresponding path may be automatically read.

In addition, the information about the capturing conditions of the image may include information, such as an exposure time at which the time-series image set is captured, a time interval for capturing the time-series images, a total number of images, a total imaging time, an aperture state, a position of a focus, a temperature of a place at which the image is captured, a date of capturing, a filter used for lighting or a lens, and information about a camera used to capture the image.

According to the embodiment of the present invention as described above, the GUI providing method for time-series image analysis provides a GUI which allows time-sequentially formed images to be easily analyzed, thereby improving user's convenience.

Although the preferred embodiments of the present invention have been disclosed for illustrative purposes, those skilled in the art should appreciate that various modifications, additions, and substitutions are possible without departing from the scope and spirit of the invention as disclosed in the accompanying claims.

## Claims

1. A graphical user interface (GUI) providing method for time-series image analysis, comprising:
reading, by a controller, at least one set of time-series images;
displaying each set of images read by the controller on a window; and
providing, by the controller, a GUI for receiving a dynamics analysis setting from a user.

2. The GUI providing method of claim 1, wherein the displaying of each set of images on the window includes displaying, by the controller, a scrollbar which allows one set of images to be sequentially displayed on one window, and
the providing of the GUI includes:
displaying, by the controller, a frame number of a currently displayed image;
displaying, by the controller, an item for designating a section to be analyzed; and
when the section to be analyzed is designated, changing, by the controller, a position of a pointer which indicates a start point and an end point of the scrollbar according to the designated section and displaying the pointer.

3. The GUI providing method of claim 1, wherein the displaying of each set of images on the window includes displaying, by the controller, a scrollbar which allows one set of images to be sequentially displayed on one window, and
the providing of the GUI includes:
displaying, by the controller, a pointer which indicates a start point and an end point of the scrollbar and,
when a position of the pointer is changed, changing, by the controller, a section to be analyzed according to the changed position of the pointer.

4. The GUI providing method of claim 1, wherein the providing of the GUI includes displaying, by the controller, an item for setting a time interval between frames of the images.

5. The GUI providing method of claim 1, wherein the providing of the GUI includes receiving, by the controller, a range of pixels to be analyzed among pixels of the images.

6. The GUI providing method of claim 5, wherein the receiving of the range of pixels includes receiving, by the controller, an analysis range through at least one of a pixel-based method, a region of interest (ROI)-based method, and a masking-based method.

7. The GUI providing method of claim 6, further comprising:
displaying, by the controller, a graph of an image value of a selected pixel over time when the analysis range is input through the pixel-based method in the receiving of the range of pixels.

8. The GUI providing method of claim 7, wherein the providing of the GUI includes displaying, by the controller, an item for setting whether to apply a regression analysis to the graph.

9. The GUI providing method of claim 7, wherein the providing of the GUI further includes displaying, by the controller, an item for setting normalization of the graph.

10. The GUI providing method of claim 6, wherein when the masking-based method is selected and a pixel is selected by the user in the receiving of the range of pixels, the controller sets pixels having image values whose difference with an image value of the selected pixel is less than a reference value as a mask.

11. The GUI providing method of claim 10, wherein when the reference value is changed, the controller changes an area of the mask according to the changed reference value, and the reference value is changeable via scrolling a mouse wheel or a reference value setting item.

12. The GUI providing method of claim 10, wherein two or more masks having different reference values are settable and an area of each mask is changeable according to an input of the user.

13. The GUI providing method of claim 6, wherein the ROI is shaped as one of a circle, an ellipse, a polygon, and a symmetrical form.

14. The GUI providing method of claim 6, further comprising:
determining, by the controller, an analysis area to be analyzed on the basis of an ROI or a mask when the analysis range is input through the ROI-based method or the masking-based method in the receiving of the range of pixels; and
displaying, by the controller, a dynamics calculation result for the determined analysis area.

15. The GUI providing method of claim 14, wherein in the determining of the analysis area, when a mask exist, the controller determines the mask area as the analysis area, and, when no mask exists, determines the ROI as the analysis area.

16. The GUI providing method of claim 14, wherein in the determining of the analysis area, when both a mask and an ROI exist, the controller sets a new ROI based on the mask and/or the ROI.

17. The GUI providing method of claim 16, wherein in the determining of the analysis area, when a plurality of ROIs exist, the controller sets pixels corresponding to an intersection between each of the ROIs and the mask as a plurality of new ROIs.

18. The GUI providing method of claim 14, wherein the displaying of the dynamics calculation result includes displaying, by the controller, a color map for the analysis area.

19. The GUI providing method of claim 14, wherein the displaying of the dynamics calculation result includes, when an ROI exists, displaying, by the controller, a feature value calculated within the ROI.

20. The GUI providing method of claim 14, wherein the displaying of the dynamics calculation result includes, when an ROI exists, displaying, by the controller, at least one of a maximum value, a minimum value, a mean value, and a standard deviation of a feature value calculated within the ROI.

21. The GUI providing method of claim 6, wherein the providing of the GUI further includes, when a plurality of ROIs are input, displaying, by the controller, an item for specifying a name of each of the ROIs in the receiving of the range of pixels.

22. The GUI providing method of claim 1, further comprising:
storing information about images read by the controller and data analyzed through the provided GUI,
wherein in the storing of the information and the data, the controller stores the information about the images and the analyzed data in a form of a single file.

23. The GUI providing method of claim 22, wherein the information about the images includes at least one of information about the read images, a storage path of the images, and information about capturing conditions of the images.
